# EUROPEAN PATENT APPLICATION

(11) **EP 4 736 930 A1**
(43) Date of publication of application: **06.05.2026**
(21) Application number: 24830594.8
(22) Date of filing: 19.06.2024
(51) Int. Cl.: A61M 25/10, A61B 17/12, A61M 39/06

(54) **CATHETER INSTRUMENT AND HEART FAILURE TREATMENT INSTRUMENT**

(30) Priority: 27.06.2023 CN 202310773544
(71) Applicant: Pulnovo Medical Pte. Ltd., Singapore 189675 (SG)
(72) Inventor: SUN, Shiche, Wuxi, Jiangsu 214191 (CN); CHEN, Mengxuan, Wuxi, Jiangsu 214191 (CN); TANG, Jian, Wuxi, Jiangsu 214191 (CN); BAI, Zhongai, Wuxi, Jiangsu 214191 (CN); SHI, Zhengmin, Wuxi, Jiangsu 214191 (CN)
(74) Representative: Mewburn Ellis LLP
(86) International application number: PCT/CN2024/100175
(87) International publication number: WO 2025/001948

(57) **Abstract**

A catheter device and a heart failure treatment device are provided. The catheter instrument comprises: a catheter body having a device pass-through lumen, a balloon catheter pass-through lumen, a balloon inflation lumen, and a pressure monitoring lumen that are not in communication with each other; a balloon sealedly coupled to an outer wall of the catheter body and connected to a distal end of the balloon inflation lumen; an attachment seat coupled to a proximal end of the catheter body and comprising a port for communication with the device pass-through lumen, a first side port for connection with the balloon catheter pass-through lumen, a second side port for connection with the balloon inflation lumen, and a third side port for connection with the pressure monitoring lumen. the balloon inflation lumen, a second side port in communication with the balloon catheter lumen, and a third side port in communication with the pressure monitoring lumen, wherein the port is for passage of a diagnostic instrument and the first side port is for passage of a balloon catheter; a first side branch for inputting a balloon inflation medium in communication with the second side port; and a second side branch for inputting a pressure monitoring medium in communication with the third side port.

## Description

### Technical field

The present disclosure relates to the technical field of medical devices and, in particular, to a catheterization device and a heart failure treatment device.

### Background technology

Heart failure is a series of symptoms that occur when the heart is not able to pump enough blood out of the heart due to a malfunction of the heart, resulting in a lack of blood perfusion. Heart failure is generally categorized into acute heart failure and chronic heart failure.

Acute heart failure is most common in acute left heart failure, which is a clinical syndrome that occurs due to acute myocardial damage or increased cardiac load, resulting in a sudden drop in acute cardiac output, elevated pressure in the pulmonary circulation, increased peripheral circulatory resistance, and subsequent congestion in the pulmonary circulation with acute pulmonary stasis, pulmonary edema, and may be accompanied by inadequate perfusion of tissues and organs and cardiogenic shock.

Chronic heart failure is categorized into right heart failure and left heart failure. In right heart failure, the ejection capacity of the right ventricle decreases and more blood remains in the right heart, resulting in increased pressure in the right heart and laborious flow of blood into the right heart. Since the blood flowing into the right heart is body circulation blood, there will be body circulation stagnation, and since the lower limbs are in the lower part of the body, the lower limbs stagnation is the most serious. When water passes from the blood vessels of the lower extremities into the surrounding tissues, edema of the lower extremities results. In left heart failure, the inflow of blood into the left heart from the pulmonary circulation leads to an increase in left atrial pressure and pulmonary venous pressure, which in turn leads to pulmonary bruising and pulmonary edema.

In related techniques, depending on the different conditions of acute heart failure, the initial treatment is oxygen, diuretics and cardiotonic agents administered intravenously; if the condition remains unrelieved, vasoactive drugs are applied to dilate and constrict the blood vessels; if the condition is severe, with a sustained decrease in blood pressure or even cardiogenic shock, hemodynamic monitoring and emergency nonpharmacological treatments are required.

There are limitations to the treatment modalities in the relevant techniques; for example, some patients respond well to intravenous diuretic therapy, but others do not. This is because, when there is too much fluid in the body, most of the excess fluid is not actually present in the lumen of the blood vessels, but in the interstitial space around the cells, a tissue called the interstitium. In order to adequately reduce congestion, the excess fluid in the interstitium of the cells first needs to be moved into the blood vessels. In the healthy body, this function is accomplished through drainage by the lymphatic system, which actively drains the fluid into the large veins above the heart. However, in patients with acute heart failure, the pressure in the large veins can be very high, which slows or even prevents the flow of lymphatic fluid into the large veins, thus impeding the process of relieving congestion. Incomplete diuresis (i.e., fluid retention) was found to be a strong predictor of rehospitalization and death, with up to half of acute heart failure patients discharged from the hospital without being completely "diuresed," resulting in about 25% of patients being readmitted to the hospital within one month, and about half of patients being readmitted within six months.

How to improve the effectiveness of heart failure treatment is a technical problem that needs to be solved.

### Content of the invention

Embodiments of the present disclosure provide a catheterization device and a heart failure treatment device that can be used to treat acute congestive heart failure and can achieve a good prognosis.

According to an aspect of the present disclosure, there is provided a catheter instrument comprising a catheter body having a device pass-through lumen, a balloon catheter pass-through lumen, a balloon inflation lumen, and a pressure monitoring lumen that are not in communication with each other; a balloon that is sealingly connected to an outer wall of the catheter body and connected to a distal end of the balloon inflation lumen; an attachment seat that is connected to the proximal end of the catheter body and comprises a port in communication with the device pass-through lumen, a first side port in communication with the balloon catheter through the lumen, a first side port in communication with the balloon inflation lumen, a second side port in communication with the balloon inflation lumen, and a third side port in communication with the pressure monitoring lumen, wherein the port is for passage of a diagnostic and therapeutic instrument and the first side port is for passage of a balloon catheter; a first side branch for input of balloon inflation medium in communication with the second side port; and a second side branch for input of a pressure monitoring medium in communication with the third side port.

In some embodiments, the catheter body is provided with at least one through-hole that connects the balloon pressurized lumen to the balloon, the balloon being a compliant wall-like balloon that fits against an outer wall of the catheter body in a contracted state.

In some embodiments, the material of the balloon includes at least one of silicone, latex, polyurethane, and polyurethane.

In some embodiments, the instrument pass-through lumen, the balloon catheter pass-through lumen, the balloon inflation lumen, and the pressure monitoring lumen are each circularly perforated, wherein the center of a cross-section of the instrument pass-through lumen, the center of a cross-section of the catheter body, and the center of a cross-section of the balloon catheter pass-through lumen are disposed sequentially along a first straight line direction; the center of a cross-section of the balloon inflation lumen and the center of a cross-section of the pressure monitoring lumen are disposed sequentially along a the center of the cross-section of the balloon pressure-filling lumen and the center of the cross-section of the pressure-monitoring lumen are disposed sequentially along a second linear direction orthogonal to the first linear direction.

In some embodiments, the aperture *R₁* of the lumen through which the device passes, the aperture *R_{(2) of}* the lumen through which the balloon catheter passes, the aperture *R₃* of the lumen through which the balloon is inflated, and the aperture *R₄* of the lumen through which the pressure is monitored, satisfy the following: *r₁* > *ᵣ₍₂₎* > *ᵣ₍₃₎*= *r₄*.

In some embodiments, the catheter body includes a main body portion and a tip portion coupled to a distal end of the main body portion, wherein the instrument leads to a side of the tip portion through the lumen and a distal end of the pressure monitoring lumen, and the balloon catheter leads to an end face of the tip portion through the distal end of the lumen.

In some embodiments, the catheter instrument further comprises: a first developing element disposed on an outer wall of the body portion; and/or a second developing element disposed on a side of the tip portion.

In some embodiments, the catheterization device further comprises: a first gland disposed at the port, and a first hemostatic valve disposed within the port and secured by the first gland; and a second gland disposed at the first side port, and a second hemostatic valve disposed within the first side port and secured by the second gland.

In some embodiments, the first hemostatic valve is a radial compression hemostatic valve and the second hemostatic valve is a cross-cut hemostatic valve.

In some embodiments, a luer fitting tee is provided at the proximal end of each of the first side branch and the second side branch.

In some embodiments, the diagnostic device includes an examination catheter, a floating catheter, or an ablation catheter.

According to an aspect of the present disclosure, there is provided a heart failure treatment device comprising a catheterization device of the preceding aspect.

According to one or more embodiments of the present disclosure, the veins may be blocked intermittently using the balloon of the catheterization device and the balloon of the balloon catheter passing through the catheterization device, thereby reducing the return of venous blood to the heart, decreasing the preload on the heart, and allowing for a reduction in ventricular wall stress. During or before and after treatment, the instrument can be passed through the lumen to the diagnostic device for relevant testing and treatment, and the intravascular pressure can be monitored through the pressure-monitoring lumen. Catheterization devices are used in the treatment of acute congestive heart failure, and can obtain a good prognosis.

These and other aspects of the present disclosure will be clearly understood and will be elucidated with reference to the embodiments described in the following.

### illustrate

In the following description of exemplary embodiments in conjunction with the accompanying drawings, further details, features and advantages of the present disclosure are disclosed in the accompanying drawings:
FIG. 1 is a schematic diagram of the main view structure of a catheterization device of some embodiments of the present disclosure; and
FIG. 2 is a schematic diagram of the three-dimensional structure of a catheterization instrument of some embodiments of the present disclosure.

Illustrated by the accompanying markings:
100-Catheter instruments
10-Catheter body
101-Instrumentation through lumen
102-Balloon catheter through lumen
103-Balloon pressurized
104 - Pressure monitoring lumen
11-Balloon
12-Connector
121-Port
122 - First side port
123 - Second lateral port
124 - Third side port
13 - First lateral branch
14 - Second lateral branch
105 - Through Hole
106 - Main part
107 - Tip section
15-First developing element
16 - Second developing element
17 - First gland
18 - Second gland
19 - First hemostatic valve
20 - Second hemostatic valve
21-Luer Fitting Tee

### Practical way of doing sth.

In the following, only certain exemplary embodiments are briefly described. As may be recognized by those skilled in the art, the described embodiments may be modified in a variety of different ways without departing from the spirit or scope of the present disclosure. Accordingly, the accompanying drawings and descriptions are considered to be essentially exemplary and not limiting.

Heart failure is now the leading cause of death worldwide and its incidence is increasing year by year. Heart failure is a condition in which the heart cannot pump enough blood to meet the body's metabolic needs due to a decrease in the pumping or filling capacity of the ventricles, resulting in inadequate perfusion of organs and tissues, as well as bruising of the lungs or the circulation. Heart failure is a syndrome of various heart diseases progressing to a severe stage and is often also referred to as congestive heart failure.

Reducing ventricular wall stress by decreasing cardiac preload is fundamental to the treatment of acute congestive heart failure. The Frank-Starling mechanism (a compensatory mechanism in heart failure) suggests that preload is a major determinant of cardiac output and further suggests that in patients with systolic heart failure in the presence of volume overload, reduction of volume overload may improve, or at least not decrease, cardiac output. The results of several current studies suggest that elevated cardiac filling pressures directly affect the short- and long-term prognosis of patients with heart failure.

Among the current treatments for heart failure, cardiac preload is often reduced by diuretic therapy and vasodilation. Methods to reduce cardiac congestion through specialized devices include accelerated water separation, hemodialysis, and some innovative methods such as pumps within the descending aorta. However, few devices specifically designed to reduce cardiac preload are currently available due to concerns about the potential to reduce cardiac output and systemic blood pressure.

Embodiments of the present disclosure provide a catheterization device and a heart failure treatment device that can be used to treat acute congestive heart failure and can achieve a good prognosis.

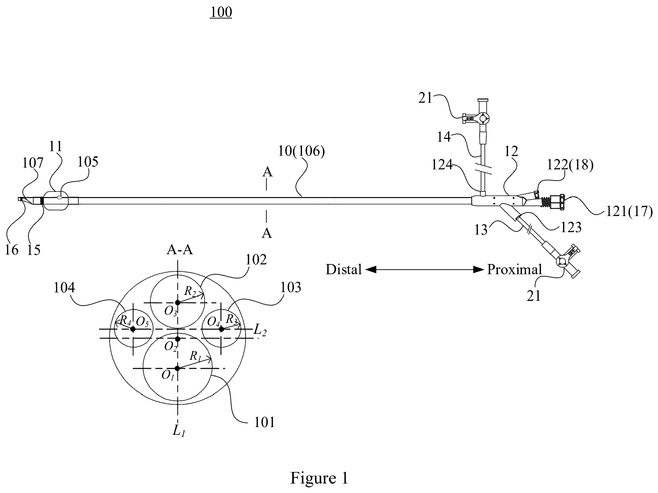

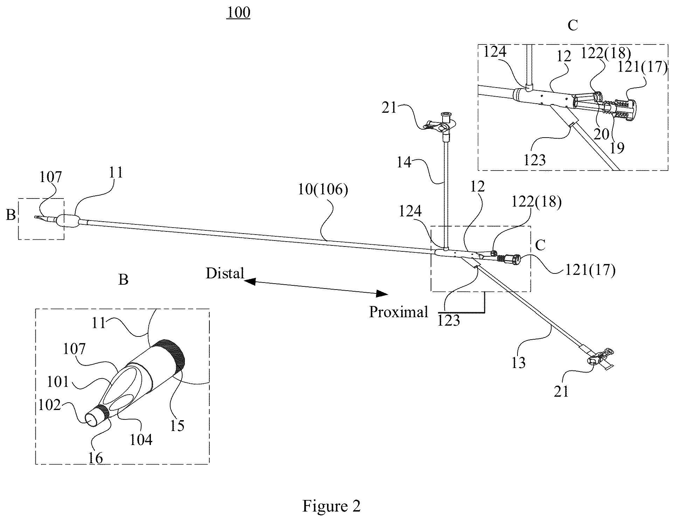

Some embodiments of the present disclosure provide catheter devices for application as heart failure treatment devices. As shown in FIGS. 1 and 2, the catheterization device 100 includes a catheter body 10, a balloon 11, an attachment seat 12, a first lateral branch 13, and a second lateral branch 14. the catheter body 10 has a device pass-through lumen 101 that is out of communication with each other, a balloon catheter pass-through lumen 102, a balloon-filled lumen 103, and a pressure-monitoring lumen 104. the balloon 11 is hermetically sealed to an outer wall of the catheter body 10 and connected to the The balloon 11 is sealed to the outer wall of the catheter body 10 and connected to the distal end of the balloon inflation lumen 103. The coupling seat 12 is connected to the proximal end of the catheter body 10 and includes a port 121 communicating with the instrument pass-through lumen 101, a first side port 122 communicating with the balloon catheter pass-through lumen 102, a second side port 123 communicating with the balloon pressure-filling lumen 103, and a third side port 124 communicating with the pressure-monitoring lumen 104, wherein the port 121 is used for penetration of a diagnostic instrument (not shown), the first side port 122 is used for penetration of an instrument (not shown), and the first side port 122 is used for penetration of an instrument (not shown). entry, and the first side port 122 is for penetration of a balloon catheter (not shown in the figures). The first side branch 13 is in communication with the second side port 123 and is used for inputting a balloon inflation medium, and the second side branch 14 is in communication with the third side port 124 and is used for inputting a pressure monitoring medium.

In embodiments of the present disclosure, the end of the catheterization device 100 or component thereof that extends closer to the operator in the direction of extension is defined as the "proximal end", and similarly, the end of the catheterization device 100 or component thereof that extends further away from the operator in the direction of extension is defined as the "distal end". ". The "proximal" and "distal" ends of a balloon catheter or other device are defined above and will not be repeated. A "side port" is defined as an orifice provided on the side surface of the component, and a "port 121" is defined as an orifice provided on the end surface of the component.

The catheterization instrument 100 of the presently disclosed embodiment is used to provide access for balloon catheters as well as other diagnostic instruments, which can be used to seal the blood vessel by filling the balloon 11 with the aid of an external pressure pump, and can also be used to monitor the intravascular pressure by means of an external pressure sensor, utilizing the pressure-monitoring lumen 104, during the procedure. The diagnostic instrument can be threaded into the instrument passage lumen 101 from the port 121 of the connection seat 12, and the balloon catheter can be threaded into the balloon catheter passage lumen 102 from the first side port 122 of the connection seat 12, and the balloon filling lumen 103 can be filled with pressure-filling medium with the aid of an external device utilizing the first side branch 13, and the pressure-monitoring medium can be filled with pressure-monitoring medium into the pressure-monitoring lumen 104 utilizing the second side branch 14. In some embodiments, a luer fitting tee 21 is provided at the proximal end of each of the first side branch tube 13 and the second side branch tube 14, thereby allowing a plurality of mutually compatible fluids to be managed using the same line.

In some embodiments of the present disclosure, the diagnostic and therapeutic instrument may be an inspection catheter, a floating catheter, or an ablation catheter, etc., and the pressure-monitoring medium may be, for example, heparin saline, etc., and the present disclosure does not make specific limitations thereon, and the selection may be made according to the actual treatment plan.

After the catheterization device 100 is placed in the body, a balloon catheter and/or a diagnostic device may be placed, guided by the catheterization device 100 and associated guide member, so that the blood vessel may be occluded using the balloon 11 of the catheterization device 100 and the balloon of the balloon catheter, and the diagnostic device may be used to carry out the associated diagnostic operation to monitor intravascular pressure by means of pressure monitoring of the lumen 104, which is directed to the distal end of the catheterization device 100 and leads into the blood vessel. The intravascular pressure is monitored. In some embodiments of the present disclosure, the process of placing the catheterization device 100 and the balloon catheter in the human body is in the following order: performing a puncture using a puncture needle → placing a short guidewire in the puncture needle → withdrawing the puncture needle → placing the catheterization device 100 using the short guidewire as a guide → withdrawing the short guidewire → placing a long guidewire into the balloon catheter of the catheterization device 100 through the lumen 102 → placing the balloon catheter using the long guidewire as a guide → withdrawing the long guidewire. pulling out the long guidewire. In this regard, the short guidewire and the long guidewire are used as intermediate auxiliary instruments when placing the catheterization instrument 100 and the balloon catheter.

According to the design of embodiments of the present disclosure, the balloon 11 of the catheterization device 100 and the balloon intermittently passing through the balloon catheter of the catheterization device 100 may be utilized to seal the veins, thereby reducing the return of venous blood to the heart, reducing the preload on the heart, and allowing for a reduction in ventricular wall stress. During or before and after treatment, the instrument can be passed through the lumen 101 for diagnostic and therapeutic instruments for relevant testing and treatment, and the intravascular pressure can be monitored through the pressure monitoring lumen 104. The catheterization device 100 of the presently disclosed embodiments can be used to treat acute congestive heart failure and can achieve good prognostic results.

The cross-sectional shape and arrangement of each lumen of the catheter body 10 is not limited. As shown in FIG. 1, in some embodiments of the present disclosure, the instrument pass-through lumen 101, the balloon catheter pass-through lumen 102, the balloon pressure-filling lumen 103, and the pressure-monitoring lumen 104 are each in the shape of a circular hole, wherein the center of a circle *O₁* of the cross-section of the instrument pass-through lumen 101, the center of a circle *O₂* of the cross-section of the catheter body 10, and the center of a circle *O₍₃₎* are disposed sequentially along the first linear direction *L₁*, and the center of circle *O₄* of the cross-section of the balloon pressure-filling lumen 103 and the center of circle *O₅* of the cross-section of the pressure-monitoring lumen 104 are disposed sequentially along the second linear direction *L₂* orthogonal to the first linear direction *L₁*.

Since the instrument passes through the lumen 101 to be passed into the diagnostic and therapeutic instrument, the aperture thereof is designed to be relatively large in order to enable it to pass through smoothly. The aperture diameter of the balloon catheter passage lumen 102 may be designed accordingly to the outer diameter of the balloon of the balloon catheter in the contracted state, so as to enable smooth passage of the balloon catheter, and the balloon inflation lumen 103 and the pressure monitoring lumen 104 may be disposed on both sides of the instrument passage lumen 101 and the balloon catheter passage lumen 102. In some embodiments, the aperture *R₁* of the device passage lumen 101, the aperture *R₂* of the balloon catheter passage lumen 102, the aperture *R₃* of the balloon-filled lumen 103, and the aperture *R₄* of the pressure-monitoring lumen 104, satisfy the following: *r₁ > ᵣ₍₂₎> ᵣ₍₃₎= r₄*.

As shown in FIG. 1, in some embodiments of the present disclosure, the catheter body 10 is provided with at least one through-hole 105 (e.g., a hole may be made directly in the outer wall of the catheter body 10 to form the at least one through-hole 105) connecting the balloon pressure-filled lumen 103 to the balloon 11, and the balloon 11 is a compliant tubular-wall shaped balloon that is in a constricted state against the outer wall of the catheter body 10.

The proximal inner wall and distal inner wall of the compliant wall-like balloon may be sealably bonded to the outer wall of the catheter body 10, thereby sealing the balloon 11.

The balloon 11 is made of a compliant material, which may include, for example, at least one of silicone, latex, polyurethane, and polyimide ester, such that a larger expansion diameter can be obtained at lower pressures, and the expansion diameter grows significantly as the pressure is elevated, so that it can be effectively anchored after expansion to provide reliable support in the blood vessel. In addition, the balloon 11 adopts a wall-like design, which fits the outer wall of the catheter body 10 in the contracted state, so that a smaller outer diameter can be obtained, in order to minimize the resistance to travel in the blood vessel, and reduce the harm to the human body.

As shown in FIG. 2, in some embodiments of the present disclosure, the catheter body 10 includes a main body portion 106 and a tip portion 107 connected to a distal end of the main body portion 106, wherein the instrumentation passes through a distal end of the lumen 101 and the pressure monitoring lumen 104 to the side of the tip portion 107, and the balloon catheter passes through a distal end of the lumen 102 to the end face of the tip portion 107. That is, the diagnostic instrument may pass through the side of the tip portion 107 of the catheter body 10 and the balloon catheter may pass through the end face of the tip portion 107 of the catheter body 10.

The material of the body portion 106 may, for example, comprise at least one of a polyamide, a polyether block polyamide, an acrylonitrile-butadiene-styrene terpolymer, and a polyethylene glycol p-toluene dicarboxylate. The tip portion 107 may be made of a material that is more flexible compared to the body portion 106, and may for example comprise at least one of polyurethane, polyimide ester, polyether block polyamide, and silicone. The tip portion 107 may be welded together with the body portion 106. The choice of material and shape of the tip portion 107 can be designed to reduce the resistance of the catheterization device 100 to traveling through the blood vessel, allowing for a smoother entry.

Referring to FIG. 1, in some embodiments of the present disclosure, the catheterization instrument 100 may further include a first developing element 15 disposed on the outer wall of the main body portion 106, and/or, a second developing element 16 disposed on the side of the tip portion 107. During surgery, the developing element may be detected using an external image detecting device (e.g., an X-ray detecting device), so as to accurately know its position, and to achieve precise positioning for instrumental Precise positioning of the intervention can be achieved. The material of the above-described developing element may be selected from one or more of gold, platinum, iridium, tantalum, tungsten and the like.

In embodiments of the present disclosure, the material of the connection seat 12 may include at least one of acrylonitrile-butadiene-styrene terpolymer, polyamide, polycarbonate, and polyformaldehyde. The connection seat 12 may provide access to other instruments or therapeutic media, and is made of a material that is stiff relative to the catheter body 10 so as to facilitate assembly and instrument manipulation. In addition, a hemostatic valve may be installed therein.

As shown in FIG. 2, in some embodiments of the present disclosure, the catheter instrument 100 further includes: a first gland 17 disposed in the port 121, and a first hemostatic valve 19 disposed within the port 121 and secured by the first gland 17, a second gland 18 disposed in the first side port 122, and a second hemostatic valve 20 disposed within the first side port 122 and secured by the second gland 18. The hemostatic valve can utilize its own The hemostatic valve can use its own elasticity to squeeze and seal, preventing blood from flowing out and realizing a hemostatic effect. The material of the first hemostatic valve 19 and the second hemostatic valve 20 may include, for example, at least one of silicone, latex, polyurethane, and polyurethane.

The specific structural form of the first hemostatic valve 19 and the second hemostatic valve 20 is not limited.

In some embodiments, the first hemostatic valve 19 is a radial compression hemostatic valve with an axial opening, and the first gland 17 cooperates with its periphery, so that when the first gland 17 is screwed down, it presses on the periphery of the first hemostatic valve 19, causing it to contract to close the hole, thereby providing hemostasis.

In some embodiments, the second hemostatic valve 20 is a cross-cut hemostatic valve, which is in the form of a sheet and has a cross-cut, and in the natural state, the cross-cut is in a closed state, and when the balloon catheter passes through the cross-cut, it will open the cross-cut, but because the hemostatic valve itself has elasticity, it can be squeezed and wrapped around the surface of the balloon catheter, and provide a sealing and hemostatic effect.

Embodiments of the present disclosure also provide a heart failure treatment device comprising the catheterization device 100 of any of the preceding embodiments.

The heart failure treatment device may include associated diagnostic devices, balloon catheters, pumps, and pressure transducers. The heart failure therapeutic device may be used in a venous occlusion regimen, allowing for a favorable prognosis for the treatment of acute congestive heart failure.

Examples of therapeutic implementations of heart failure treatment devices according to some embodiments of the present disclosure are as follows:
First, a channel is established in the femoral vein by means of a puncture kit and a guidewire is placed; then, the catheterization device 100 is introduced into the body along the guidewire from the femoral vein, and the balloon 11 of the catheterization device 100 is positioned under contrast to the inferior vena cava, which is positioned underneath the renal vein, to secure the catheterization device 100;

Then, a guidewire is placed in the balloon catheter through the lumen 102 of the catheterization apparatus 100, so that the guidewire passes upwardly along the inferior vena cava through the heart, the superior vena cava, and the vein of the head and arm to the subclavian vein; then, the balloon catheter is placed to reach the designated position along the guidewire through the balloon catheter through the lumen 102, and in the process, the contrast agent may be delivered to the blood vessel through the contrast agent delivery holes that are provided with the balloon catheter, so as to enable the balloon catheter's balloon to accurately reach the blocking location.

After that, the catheterization instrument 100 and the balloon catheter are each connected to a pressure pump to start the treatment process.

During or before or after treatment, the instrument can be passed through the lumen 101 to a diagnostic device for relevant testing and treatment, the diagnostic device can be, for example, a floating catheter, an ablation catheter, etc., and in addition, the intravascular pressure can be monitored by the pressure-monitoring lumen 104 during the treatment.

It can be seen that for venous occlusion, the treatment plan can be to occlude the veins by two balloons working together, thus reducing the venous return to the heart and reducing the cardiac preload.

It is to be understood that in this specification the terms "center", "longitudinal", "transverse", "length", "width", "thickness", "top", "bottom", "bottom", "thickness", and "length" are used. ", "width", "thickness", "top", "bottom", "front", "back", "left", "right", "vertical ", "horizontal", "top", "bottom", "inside", "outer", "clockwise", "counterclockwise", "axial", " radial," "circumferential," and the like indicate orientations or positional relationships or dimensions based on those shown in the accompanying drawings, and that the use of these terms is solely for the purpose of facilitating description, and does not indicate or imply that the device or element referred to must have a particular orientation, be constructed and operated in a particular orientation. and therefore are not to be construed as a limitation on the scope of protection afforded by this public .

In addition, the terms "first", "second", "third", etc. are used only for descriptive purposes and are not to be understood as indicating or implying relative importance or implicitly specifying the number of technical features indicated. The number of technical features indicated is not to be understood as indicating or implying relative importance or implicitly specifying the number of technical features indicated. Thus, a feature defined with "first", "second", "third" may expressly or implicitly include one or more such features. In the description of the present disclosure, "more than one" means two or more, unless otherwise expressly and specifically limited.

For the purposes of this disclosure, unless otherwise expressly provided and limited, the terms "mounted", "connected", "connected", "fixed", and the like shall be broadly construed. " and the like are to be broadly construed, for example, as a fixed connection, a removable connection, or an integral part; a mechanical connection, an electrical connection, or a communication; a direct connection, an indirect connection through an intermediate medium, a connection within two elements, or an interactive relationship between two elements. For those of ordinary skill in the art, the specific meaning of the above terms in the present disclosure may be understood on a case-by-case basis.

In the present disclosure, unless otherwise expressly provided and limited, "over" or "under" the first feature of the second feature may include direct contact between the first and second features, or it may include contact between the first and second features not directly, but by means of another feature between them. The first feature is in contact with the second feature. Furthermore, the first feature being "above", "above" and "above" the second feature includes the first feature being directly above and diagonally above the second feature, or simply indicating that the first feature is horizontally higher than the second feature. above the second feature. The first feature being "below", "under", and "beneath" the second feature includes the first feature being directly below and diagonally below the second feature, or simply indicating that the first feature is horizontally smaller than the second feature. second feature.

This specification provides many different implementations or examples that can be used to realize the present disclosure. It should be understood that these different embodiments or examples are entirely exemplary and are not intended to limit the scope of protection of the present disclosure in any way. Those skilled in the art will be able to think of various variations or substitutions based on the disclosure of the specification of the present disclosure, and these should be covered by the scope of protection of the present disclosure. Therefore, the scope of protection of the present disclosure shall be subject to the scope of protection limited by the appended claims.

## Claims

1. , A catheter instrument comprising:
A catheter body having a lumen for passage of instruments not in communication with each other, a lumen for passage of a balloon catheter, a lumen for balloon inflation, and a lumen for pressure monitoring;
A balloon, sealingly connected to the outer wall of said catheter body and in communication with the distal end of said balloon pressurized lumen;
A coupling seat, coupled to a proximal end of said catheter body and comprising a port in communication with said instrument through a lumen, a first side port in communication with said balloon catheter through a lumen, a second side port in communication with said balloon pressure-filled lumen, and a third side port in communication with said pressure-monitoring lumen, wherein said port is for threading of a diagnostic instrument and said first side port is for threading of a balloon catheter
A first side branch tube for input of balloon pressurization medium in connection with said second side port; and
A second side branch for inputting a pressure monitoring medium in connection with said third side port.

2. , A catheterization device according to claim 1, wherein the
Said catheter body is provided with at least one through-hole connecting said balloon pressurized lumen to said balloon, said balloon being a compliant walled balloon which in a contracted state abuts the outer wall of said catheter body.

3. , A catheterization device according to claim 2, wherein the
The material of said balloon includes at least one of silicone, latex, polyurethane, and polyurethane.

4. , A catheterization device according to claim 1, wherein the
Said instrument pass-through lumen, said balloon catheter pass-through lumen, said balloon inflation lumen and said pressure monitoring lumen are each circularly perforated, wherein the
Said instrument is disposed sequentially in a first linear direction through the center of a circle in cross-section of the lumen, the center of a circle in cross-section of said catheter body, and the center of a circle in cross-section of said balloon catheter through the lumen;
The center of a circle of a cross-section of said balloon filling lumen and the center of a circle of a cross-section of said pressure monitoring lumen are disposed sequentially along a second linear direction orthogonal to said first linear direction.

5. , A catheterization device according to claim 4, wherein the
The aperture *R₁* of said instrument passing through the lumen, the aperture *R₂* of said balloon catheter passing through the lumen, the aperture *R₃* of said balloon inflation lumen and the aperture *R₄* of said pressure monitoring lumen satisfy: *R₁* > *_{R(2)}* > *_{R(3)}*= *R₄.*

6. , A catheterization device according to claim 1, wherein the
Said catheter body comprises a body portion and a tip portion connected to a distal end of said body portion, wherein said instrument leads to a side of said tip portion through a lumen and a distal end of said pressure monitoring lumen, and said balloon catheter leads to an end face of said tip portion through a distal end of the lumen.

7. , A catheter instrument according to claim 6, further comprising:
a first developing element disposed in an outer wall of said body portion; and/or
A second developing element provided on the side of said tip portion.

8. , A catheter instrument according to claim 1, further comprising:
a first gland provided in said port, and a first hemostatic valve provided within said port and held in place by said first gland; and
A second gland provided in said first side port, and a second hemostatic valve provided within said first side port and held in place by said second gland.

9. , A catheterization device according to claim 8, wherein the
Said first hemostatic valve is a radial compression hemostatic valve and said second hemostatic valve is a cross-cut hemostatic valve.

10. , A catheterization device according to claim 1, wherein the
A luer fitting tee is provided at the proximal end of each of said first side branch and said second side branch.

11. , A catheterization device according to any one of claims 1 to 10, wherein the
Said diagnostic instrument includes an examination catheter, a floating catheter, or an ablation catheter.

12. , A heart failure treatment device comprising: a catheterization device according to any one of claims 1 to 11.
